# EUROPÄISCHE PATENTSCHRIFT

(11) **EP 0 375 889 B1**
(45) Veröffentlichungstag und Bekanntmachung des Hinweises auf die Patenterteilung: **20.07.1994**
(21) Anmeldenummer: 89120459.6
(22) Anmeldetag: 06.11.1989
(51) Int. Cl.: C12N 15/10, C12P 19/34, C12N 15/77, C12N 1/20

(54) **Verfahren zur ortsspezifischen Mutagenese von DNA und Entwicklung von Plasmidvektoren**
Method for the site-specific mutagenesis of DNA and development of plasmid vectors
Méthode pour la mutagénèse spécifique pour un site de l'ADN et développement de vecteurs plasmidiques

(30) Priorität: 09.12.1988 DE 3841454
(43) Veröffentlichungstag der Anmeldung: 04.07.1990
(73) Patentinhaber: Degussa Aktiengesellschaft, 60311 Frankfurt (DE)
(72) Erfinder: Kassing, Friedrich, D-4830 Gütersloh (DE); Kalinowski, Jörn, D-4800 Bielefeld 1 (DE); Arnold, Walter, D-4800 Bielefeld 1 (DE); Winterfeldt, Andrea, D-3013 Barsinghausen 1 (DE); Pühler, Alfred, Prof., D-4800 Bielefeld 15 (DE); Kautz, Petra-Sabine, D-4800 Bielefeld 16 (DE); Thierbach, Georg, Dr., D-4800 Bielefeld 1 (DE)

(56) Entgegenhaltungen:
- JOURNAL OF GENERAL MICROBIOLOGY; Band 131, 1985, S. 1299-1303, SGM, Colchester, GB A.W. BIRCH et al.
- ANTIMICROBIAL AGENTS AND CHEMOTHERAPY, vol. 23, no. 3, March 1983, pp. 506-508, Am. Soc. for Microbiol., Washington, DC, US M. KONO et al.;
- PATENT ABSTRACTS OF JAPAN, vol. 9, no. 17 (C-262) (1740), January 24, 1985 & JP-A-59 166 087 (DAIICHI SEIYAKU K.K.) 19-09-1984

## Beschreibung

Die Erfindung betrifft ein Verfahren zur ortsspezifischen Mutagenese von DNA und die Entwicklung von Plasmidvektoren.

Corynebacterium glutamicum, Brevibacterium flavum und verwandte Stämme bzw. von diesen abgeleitete Mutanten sind bekannte Organismen, mit deren Hilfe L-Aminosäuren, wie z.B. Lysin und Threonin fermentativ hergestellt werden.

Für die gentechnische Stammverbesserung sind Plasmidvektoren essentielle Voraussetzung. Die Konstruktion von Plasmidvektoren für Corynebacterium bzw. Brevibacterium beruht im allgemeinen auf kryptischen Plasmiden, die in dieser Gruppe von Bakterien gefunden werden können. Beispielhaft seien genannt das Plasmid pCG1 aus Corynebacterium glutamicum ATCC31808 (US-PS 4,617,267), das Plasmid pAM330 aus Brevibacterium lactofermentum ATCC 13869 (EP-A- 0 093611) und das Plasmid pHM1519 aus Corynebacterium glutamicum ATCC 13058 (Miwa et al. 1984). Die Plasmidvektoren enthalten weiterhin mindestens eine DNA-Region, die dem Wirt Resistenz gegen ein Antibiotikum vermittelt. Beispielhaft seien genannt das Kanamycin-Resistenzgen des Transposons Tn5 (Santamaria et al. 1984), das Kanamycin-Resistenzgen des Plasmids pUB110 aus Staphylococcus aureus (EP-A- 0 093611), das Hygromycin-Resistenzgen aus Streptomyces hygroscopicus (Santamaria et al. 1987) und das Chloramphenicol-Resistenzgen aus Streptomyces acrimycini (Santamaria et al. 1987).

Plasmidvektoren für Corynebacterium und Brevibacterium können dazu benutzt werden, Gene der Biosynthese von Aminosäuren zu klonieren, das entsprechende Genprodukt bzw. Enzym in einem gesteigerten Maß zu exprimieren und dadurch die Aminosäuren-Ausscheidung zu verbessern. Beispielhaft seien genannt die Verbesserung der Ausscheidung von L-Lysin durch Corynebacterium glutamicum durch Klonierung und Überexpression des Phosphoenolpyruvat-Carboxylase-Gens von Corynebacterium glutamicum (Britisch Patentanmeldung 8821319.4).

Plasmidvektoren sind aus mehreren DNA-Regionen zusammengesetzt. Eine DNA-Region ermöglicht dem Plasmid die Replikation im entsprechenden Wirtsorganismus. Eine zweite DNA-Region vermittelt der Zelle Resistenz gegen ein Antibiotikum und kann als genetische Marke zur Selektion der plasmidtragenden Zellen einer Population benutzt werden. Eine dritte DNA-Region vermittelt Resistenz gegen ein weiteres Antibiotikum und kann als genetische Marke zur Insertionsinaktivierung genutzt werden. Bei Vorhandensein geeigneter Restriktionsschnittstellen in einer der beiden genetischen Marken kann diese jeweils zur Insertionsaktivierung genutzt werden, während die zweite Marke zur Selektion dient. Beispielhaft seien die dem Fachmann bekannten Plasmidvektoren pBR322 (Bolivar et al. 1979) und pACYC177 (Chang et al. 1978) für E.coli genannt.

Voraussetzung für die Konstruktion derartiger Plasmidvektoren sind Gene, die dem Wirt Resistenz gegen Antibiotika vermitteln. Eine weitere Voraussetzung ist das Vorhandensein singulärer Restriktionsschnittstellen in den entsprechenden Resistenzgenen der Plasmidvektoren, um diese zur Insertionsinaktivierung nutzen zu können.

Um Restriktionsschnittstellen in klonierten Antibiotika-Resistenzgenen zur Insertionsinaktivierung nutzen zu können, ist es nötig, endogene Schnittstellen im Plasmid zu entfernen. Dies kann z. B. durch Auffüllen der Schnittstelle mit Nukleosidtriphosphaten oder durch Nukleasebehandlung erfolgen. Diese Methoden sind aber nur sehr bedingt anwendbar, wenn es sich um eine in einem Gen oder in einer anderen essentiellen Region gelegene Schnittstelle handelt, weil damit ein Wechsel des Leserasters oder eine Deletion von DNA verbunden ist. Die Einführung von Punktmutationen ist nicht mit diesen Nachteilen verbunden. Eine Methode, die Punktmutationen setzt, wie die Mutagenese mit Hydroxylamin (Birch et al. 1985) birgt wiederum die Gefahr, unspezifisch mehrere Mutationen verteilt über ein ganzes Plasmid zu erzeugen.

Aufgabe der Erfindung ist ein Verfahren zur ortsspezifischen Mutagenese von DNA, um z. B. die Restriktionsschnittstellen in klonierten Antibiotika-Resistenzgenen zur Insertionsinaktivierung nutzen zu können, und die Konstruktion neuer Plasmidvektoren.

Gegenstand der Erfindung ist ein Verfahren zur ortsspezifischen Mutagenese vonDNA an den Restriktionsschnittstellen, das dadurch gekennzeichnet ist, daß man die DNA isoliert, mit einem geeigneten Restriktionsenzym spaltet, die so behandelte DNA mit einem im allgemeinen 0,5 - 2 Mol/l, bevorzugt 1 Mol/l, Hydroxylamin enthaltenden Mutageneseansatz mischt und eine ausreichende Zeit, im allgemeinen 10 bis 60 min, bevorzugt 20 bis 30 min bei erhöhter Temperatur, bevorzugt bei 65 bis 70 ^{o}C inkubiert mit der Maßgabe, daß der DNA-Doppelstrang nicht oder nur im Bereich der Schnittstelle "aufschmilzt", anschließend die DNA nach an sich bekannten Methoden aufnimmt, mit einer Ligase behandelt, einen geeigneten Mikroorganismus mit der so mutierten DNA transformiert und die Transformanten isoliert.

Die Isolierung von DNA aus Zellen von Mikroorganismen erfolgt nach den allgemein bekannten Verfahren, ebenso die Spaltung an den Restriktionsschnittstellen, die Behandlung mit Ligase und die folgenden Schritte.

Der Mutageneseansatz enthält die bei Birch et al. beschriebenen Komponenten.

Gegenstand der Erfindung ist ebenso die nach dem beschriebenen Verfahren behandelte DNA, die eine oder mehrere inserierte Restriktionsschnittstellen aufweist.
Insbesondere geeignet ist eine DNA, die aufgrund dieser Mutagenese eine oder mehrere singuläre Restriktionsschnittstellen in einem oder, falls vorhanden, mehreren Resistenzgenen und/oder dem Replikon aufweist.

Diese für eine Resistenz codierenden DNA-Abschnitte können auch von einem inserierten Transposon stammen, wie sie allgemein bekannt sind.
Bevorzugt setzt man DNA in Form von Plasmiden, Plasmidvektoren oder Phagenvektoren ein, die in den Ansprüchen der Einfachheit halber als Plasmide erscheinen.

Gegenstand der Erfindung sind ebenfalls die die mutierte DNA tragenden Mikroorganismen, unter denen besonders Stämme der Gattung Corynebacterium, Brevibacterium oder abgeleitete Aminosäuren,ausscheidenden Mutanten bevorzugt sind.

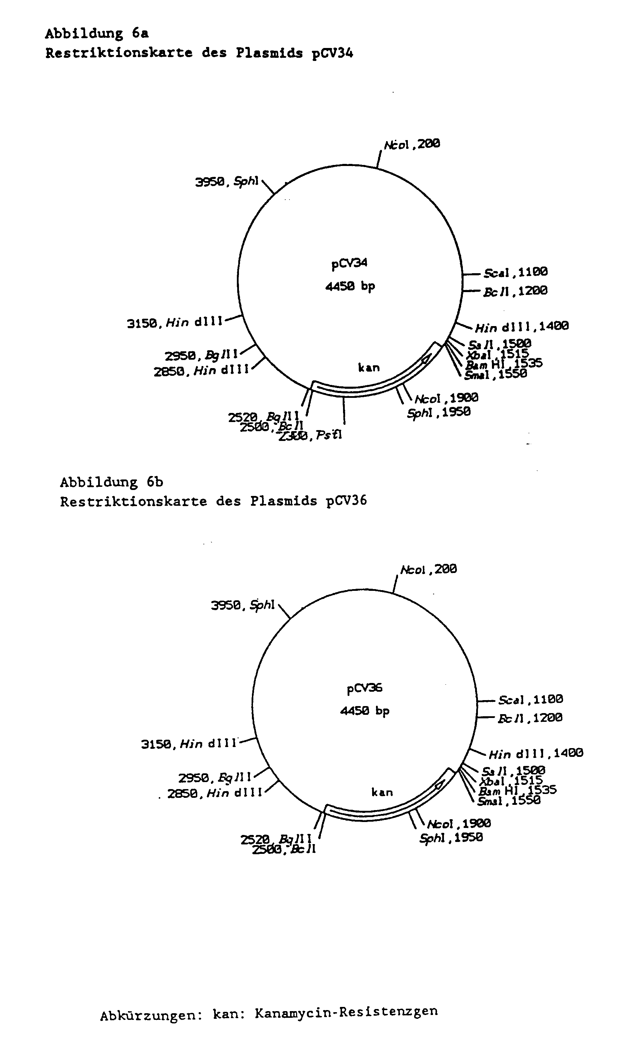

Die Erfindung bezieht sich weiterhin auf mutierte Formen von DNA, die mit Hilfe des erfindungsgemäßen Verfahrens hergestellt werden. Als Beispiel seien die Plasmide pCV34 (Abb. 6a) und pCV36 (Abb. 6b) genannt, die gegenüber dem Ausgangsplasmid eine mutierte EcoRI-Schnittstelle (pCV34) bzw. eine mutierte EcoRI- und eine mutierte PstI-Schnittstelle (pCV36) besitzen und somit mutierte Formen des pHM1519-Replikons (Miwa et al. 1984) sind.

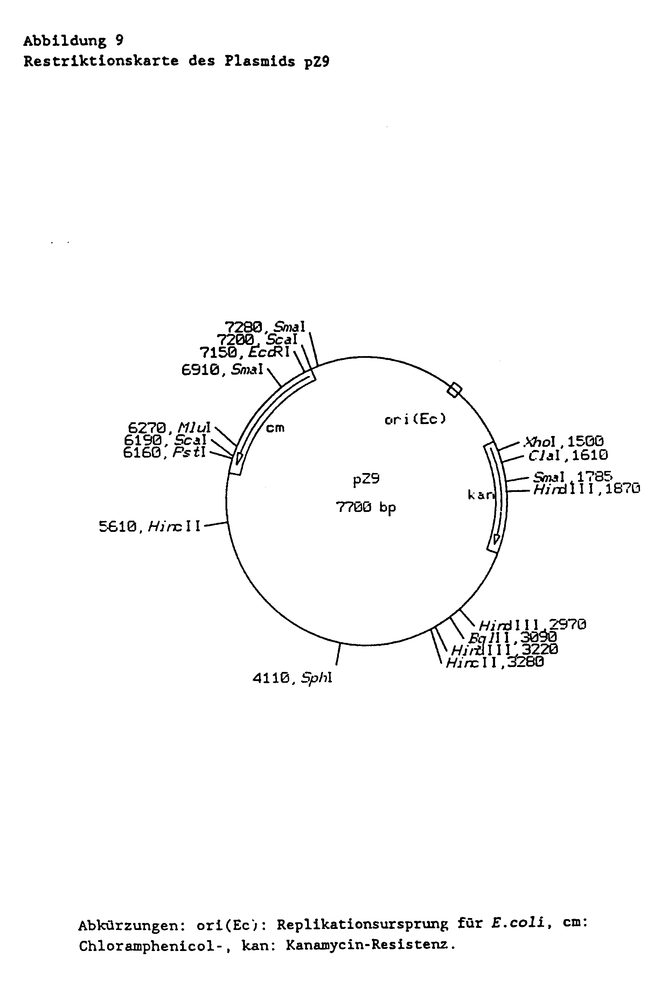

Diese Plasmide sind durch die entsprechenden Restriktionsenzyme nicht mehr spaltbar und deshalb für die Konstruktion von Plasmidvektoren den Ausgangsplasmiden vorzuziehen. Weiterhin können DNA-Fragmente in Plasmidmolekülen gegen solche ausgetauscht werden, die Mutationen tragen, welche mit dem beschriebenen Verfahren hergestellt wurden. Eine derartige Vorgehensweise ist in Beispiel 5.4 bei der Konstruktion des Plasmidvektors pZ9 (Abb. 9) gezeigt. Wirte für die Plasmide pCV34, pCV36 und pZ9 sind Stämme der Gattungen Corynebacterium und Brevibacterium sowie insbesondere von diesen abgeleitete, Aminosäuren-ausscheidende Mutanten. Für Plasmid pZ9 ist außerdem E.coli ein Wirt. Die Plasmid-stragenden Stämme der Gattungen Corynebacterium, Brevibacterium und Escherichia können nach konventionellen Methoden, wie sie dem Fachmann bekannt sind, kultiviert werden. folgende Stämme wurden bei der Deutschen Sammlung von Mikroorganismen hinterlegt:
Corynebacterium glutamicum ATCC 13032/pCV34 als DSM 5025, Corynebacterium glutamicum ATCC 13032/pCV36 als DSM 5026 und Escherichia coli DH5 /pZ9 als DSM 4939.

Ein weiterer Bestandteil der Erfindung ist der Einsatz von Antibiotika-Resistenzgenen aus Corynebacterium xerosis für die Konstruktion von Plasmidvektoren, die im Gram-negativen und Gram-positiven Bakterien und insbesondere in Aminosäure-ausscheidenden Stämmen der Arten Corynebacterium glutamicum, Brevibacterium flavum und verwandten Arten replizieren.

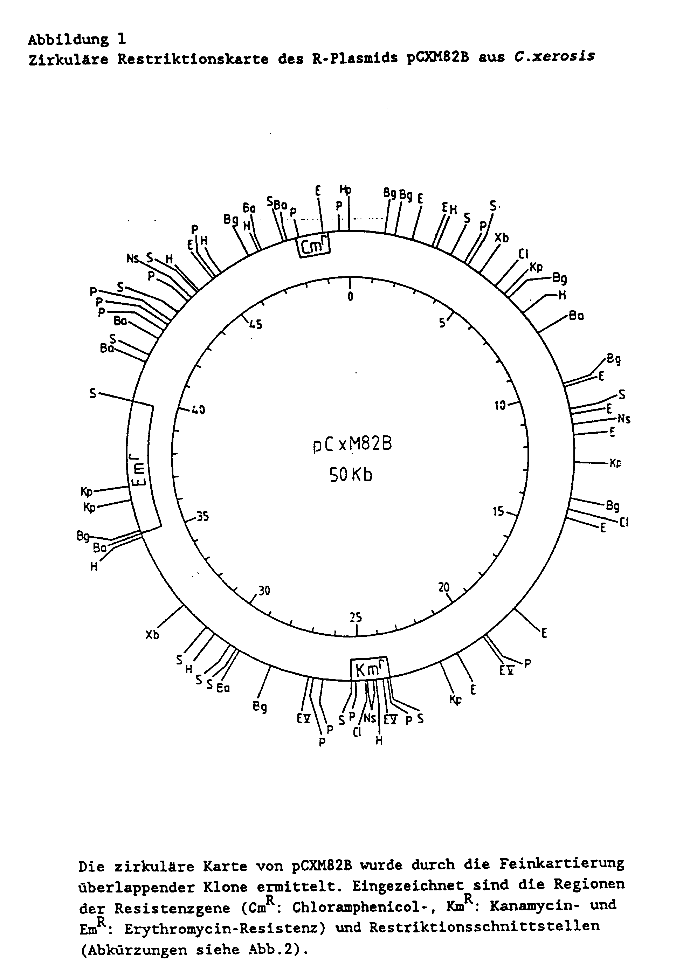

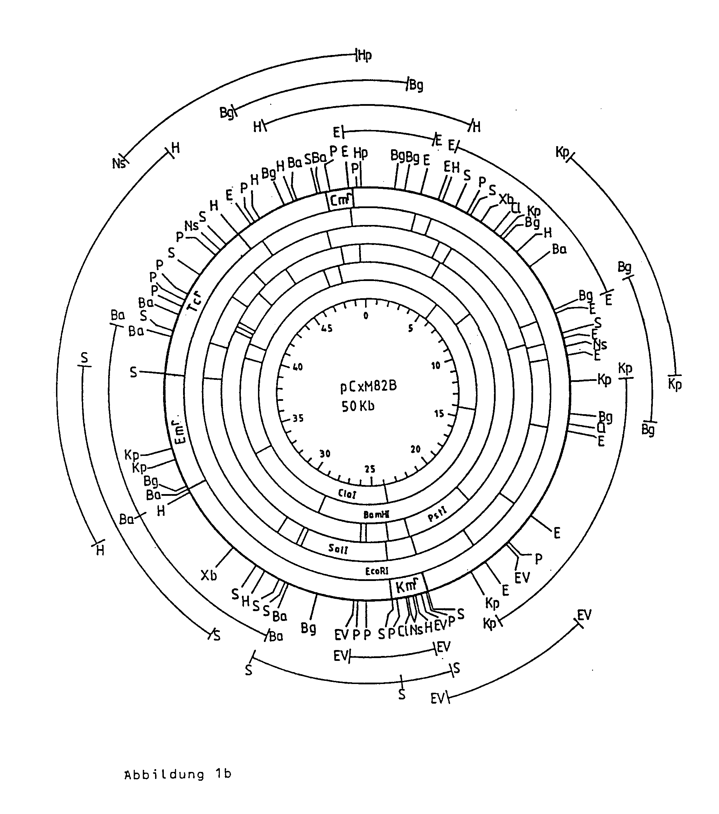

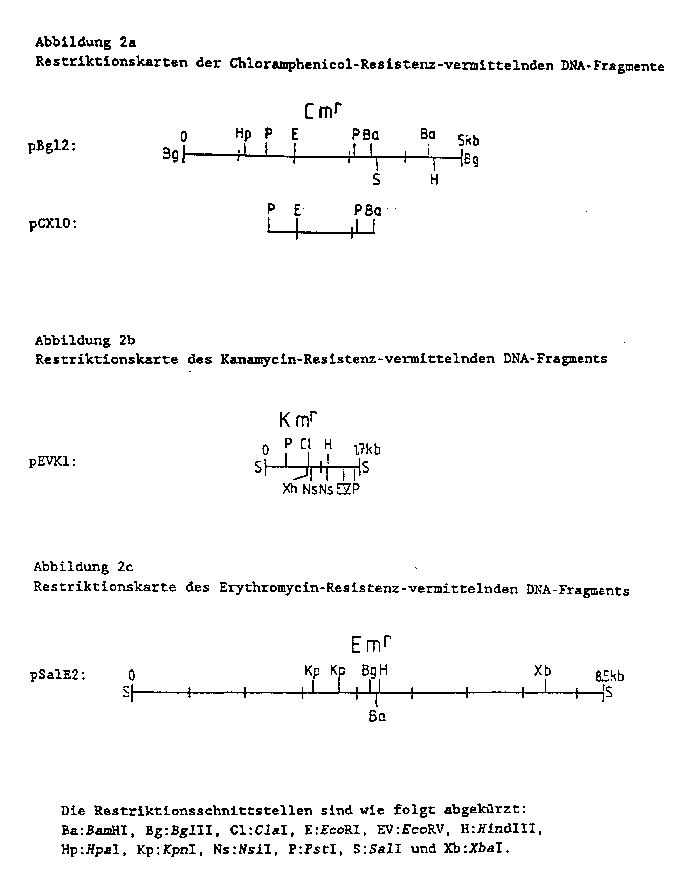

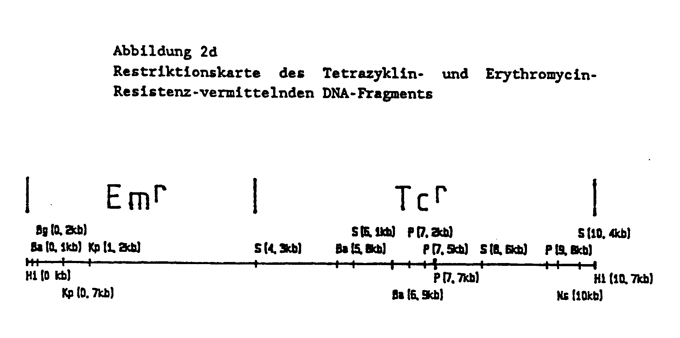

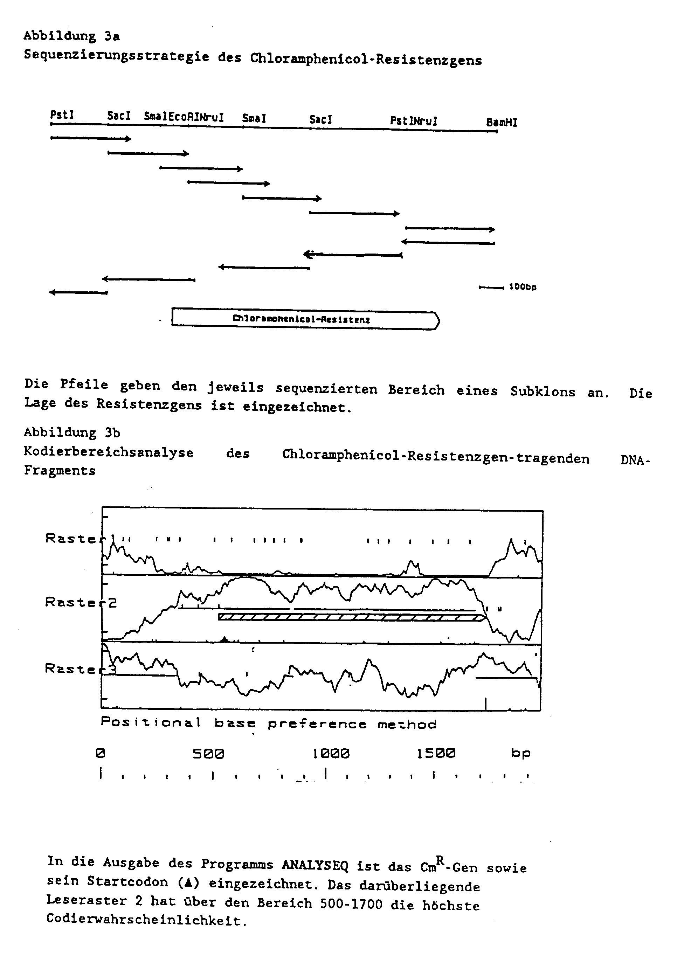

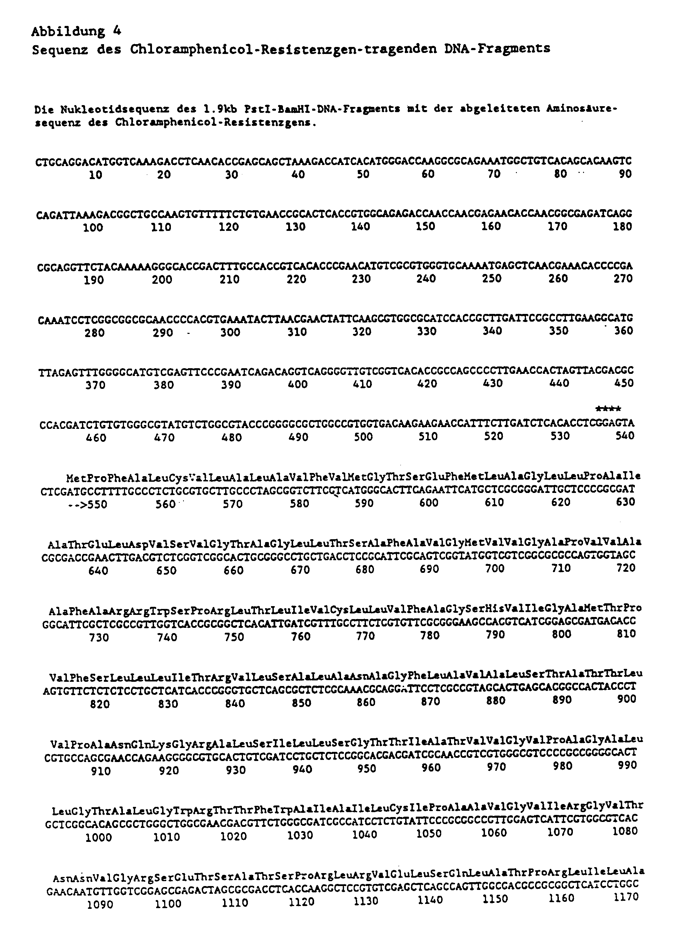

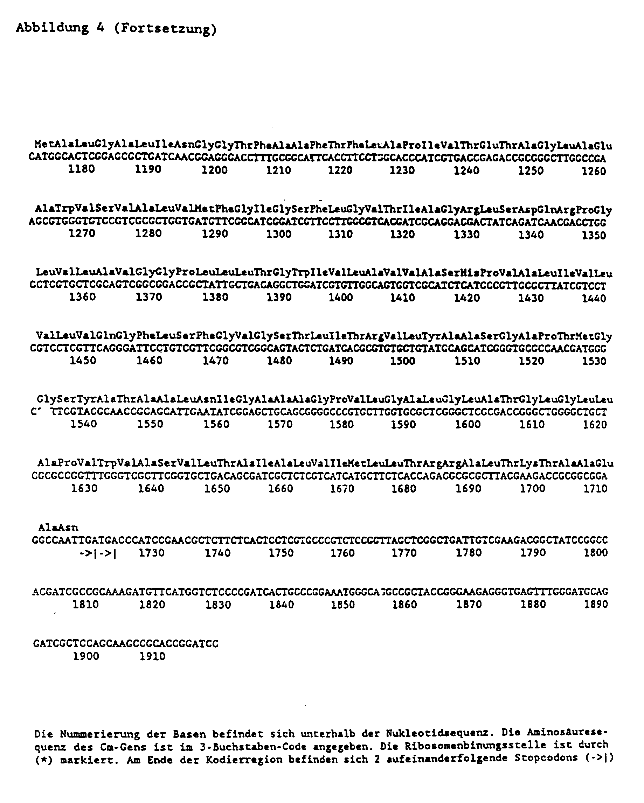

Ein in dem von Kono et al. (1983) isolierten Stamm von Corynebacterium xerosis M82B enthaltenes, etwa 50 Kilobasenpaare (kb) umfassendes Resistenzplasmid wurde mittels einer modifizierten Lyse nach Birnboim und Doly (1982) isoliert und als pCXM82B bezeichnet. Nach Verdauung der Plasmid-DNA mit Restriktionsenzymen konnte das Plasmid durch Vergleich der in Einzel- und Doppelverdauung erhaltenen DNA-Fragmente charakterisiert werden. Nach Klonierung überlappender DNA-Fragmente wurden die in den Abb. 1a und 1b dargestellten Restriktionskarten gefunden. Aus dem Plasmid pCXM82B wurden unter Zuhilfenahme des dem Fachmann bekannten E.coli-Vektors pUC19 DNA-Fragmente isoliert, welche Gene tragen,d ie dem Wirt Resistenz gegen die Antibiotika Chloramphenicol, Kanamycin und Erythromycin verleihen. Das Chloramphenicol-Resistenzgen liegt auf einem 5 kb langen BglII-DNA-Fragmente (Abb. 2a). Das Kanamycin-Resistenzgen liegt auf einem 1,7 kb langen SalI-DNA-Fragment (Abb. 2b). Das Erythromycin-Resistenzgen liegt auf einem 8,5 kb langen SalI-DNA-Fragment (Abb. 2c). Ein Tetrazyklin-Resistenzgen, das nicht in E.coli, aber in C.glutamicum zur Ausprägung einer Resistenz führt, konnte nach Fusion eines das Gen (Abb. 2d) enthaltenden pUC19-Plasmids mit einem Corynebacterien-Replikon und Transformation in C.glutamicum identifiziert werden. Der Stamm Corynebacterium glutamicum ATCC 13032 mit dem Pendelvektor p2Hi4S (Tc^{R}) wurde bei der Deutschen Stammsammlung von Mikroorganismen als DSM 5396 hinterlegt. Die beschriebenen DNA-Fragmente mit den entsprechenden Resistenzgenen können von jedem Fachmann aus dem bei der Deutschen Stammsammlung von Mikroorganismen unter DSM 5021 hinterlegten Stamm von Corynebacterium xerosis M82B isoliert werden. Die DNA-Sequenz des Chloramphenicol-Resistenz vermittelten DNA-Fragments wurde als Bestandteil der Erfindung bestimmt. Die Analyse des Kodierbereichs ist in Abb. 3b und die DNA-Sequenz in Abb. 4 dargestellt. Das Strukturgen umfaßt 1173 Basenpaare mit einem ATG-Codon am Beginn und zwei TGA-Codonen am Ende des Strukturgens.

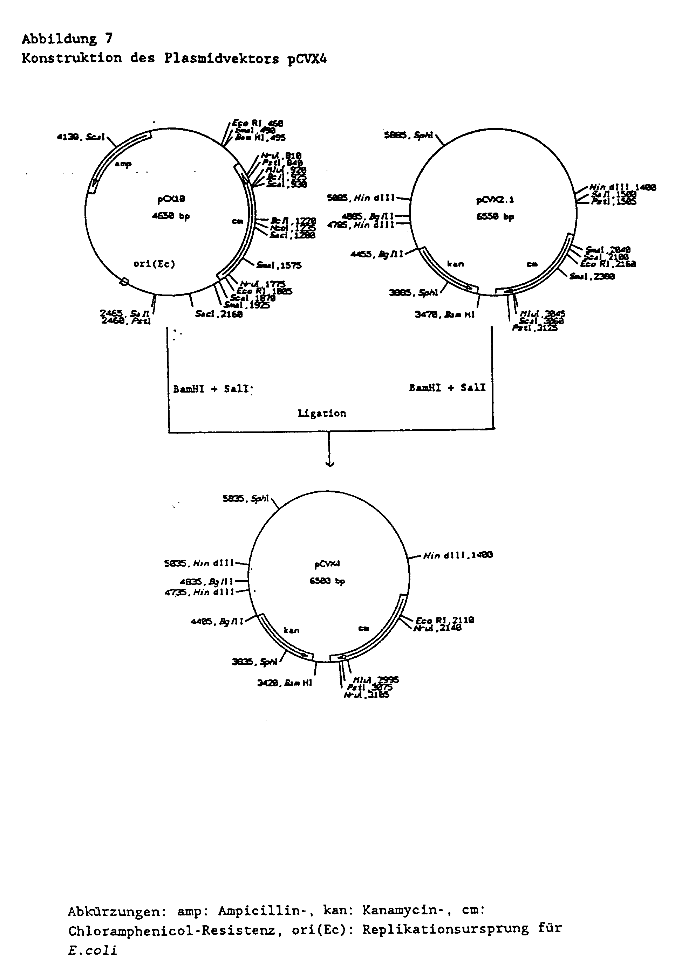

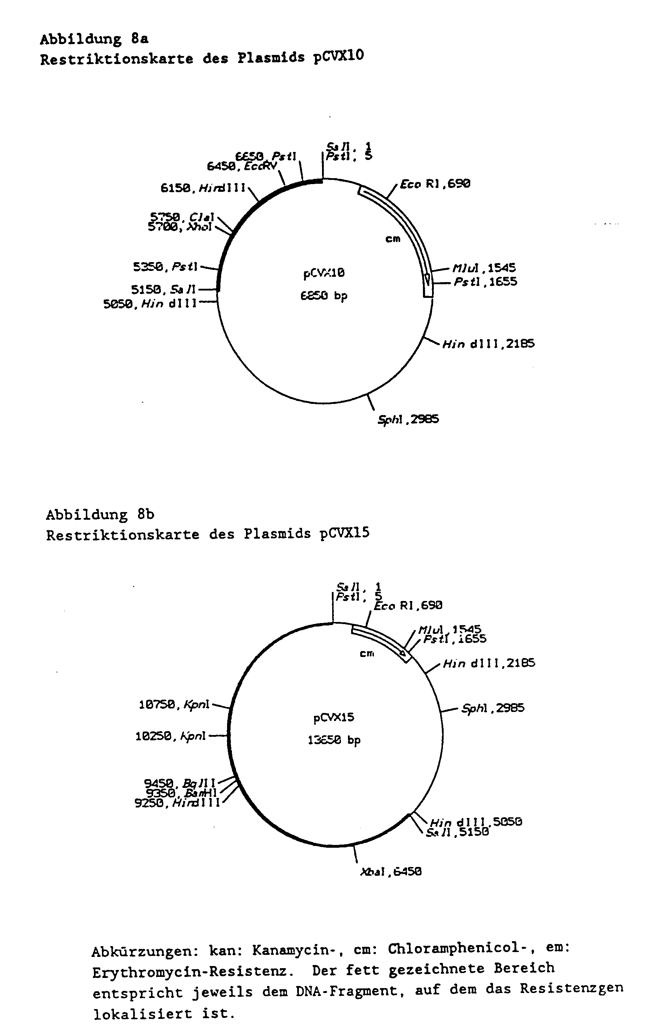

Als Bestandteil der Erfindung wurden weiterhin, ausgehend von den oben beschriebenen Resistenzgenen aus Corynebacterium xerosis, Plasmidvektoren konstruiiert, die in Aminosäuren-ausscheidenden Stämmen der Arten Corynebacterium glutamicum und Brevibacterium flavum bzw. verwandten Arten replizieren. So wurde, ausgehend von dem oben beschriebenen Plasmid pCV36, unter Verwendung des Chloramphenicol-Resistenzgens der in Abb. 7 dargestellte Plasmidvektor pCVX4 konstruiert, der neben dem Chloramphenicol-Resistenzgen das Kanamycin-Resistenzgen des Transposons Tn5 trägt. Das Plasmid pCVX4 hat eine Länge von 6,5 kb und bietet die Möglichkeit zur Insertionsinaktivierung durch die singulären Restriktionsschnittstellen EcoRI, PstI und MluI im Chloramphenicol-Resistenzgen. Weiterhin wurde der Plasmidvektor pCVX10 (Abb. 8a) konstruiert, der eine Länge von 7 kb hat und das Kanamycin-Resistenzgen von Corynebacterium xerosis mit den singulären Restriktionsschnittstellen XhoI und ClaI sowie das oben beschriebene Chloramphenicol-Resistenzgen von Corynebacterium xerosis trägt. Schließlich wurde der in Abb. 8b dargestellte Plasmidvektor pCVX15 konstruiert, der eine Länge von 13,8 kb hat und das Erythromycin-Resistenzgen sowie das oben beschriebne Chloramphenicol-Resistenzgen von Corynebacterium xerosis trägt.

Folgene Stämme wurden bei der Deutschen Stammsammlung von Mikroorganismen hinterlegt
Corynebacterium glutamicum ATCC 13032/pCVX4 als DSM 5022, Corynebacterium glutamicum ATCC 13032/pCVX10 als DSM 5023, Corynebacterium glutamicum ATCC 13032/pCVX15 als DSM 5024.

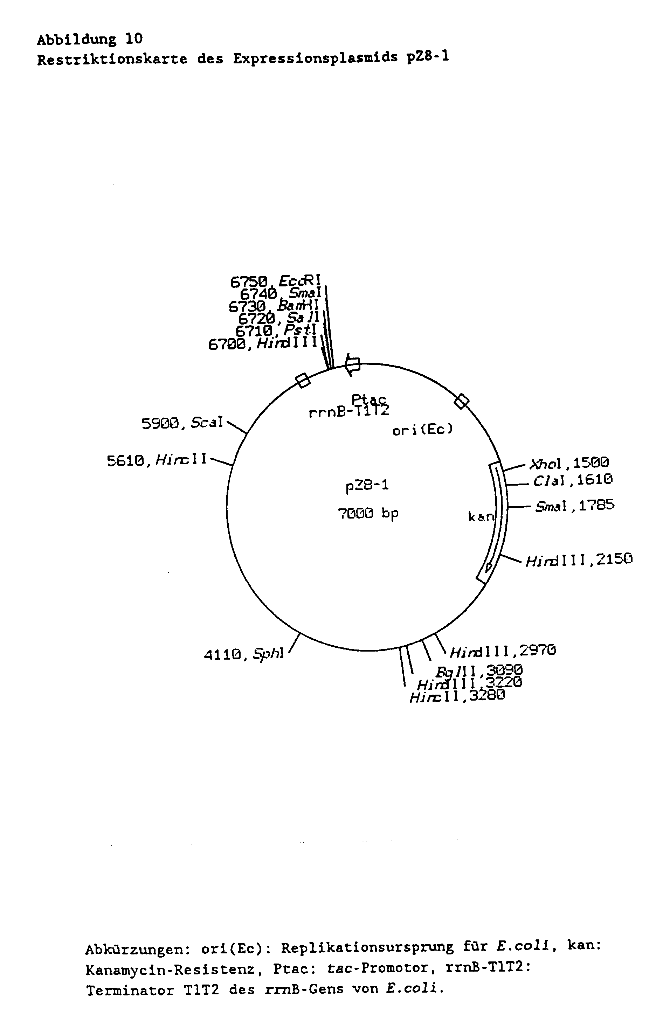

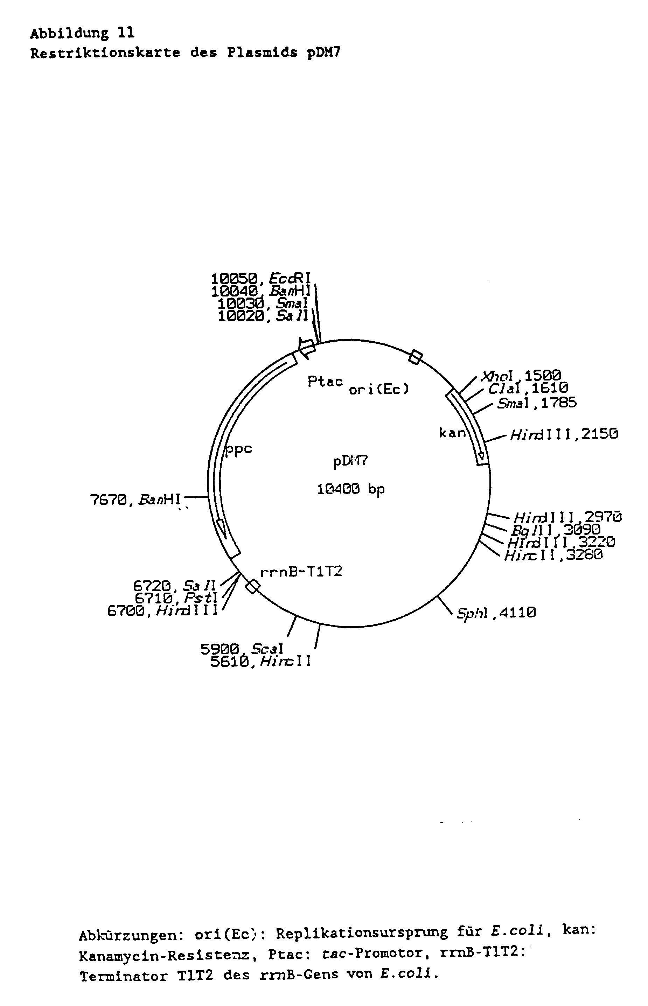

Weiterhin ist der vorteilhafte Expressionsvektor pZ8-1, der in Abb. 10 dargestellt ist, ein Bestandteil der Erfindung. Plasmid pZ8-1 hat eine Länge von 7,0 kb und trägt die nach dem oben beschriebenen neuen Verfahren der Hydroxylamin-Mutagenese hergestellte mutierte Form des pHM1519-Replikons, welche während der Konstruktion durch Austausch eines DNA-Fragmentes in das Plasmid eingeführt wurde. Im besonderen trägt das Plasmid pZ8-1 den tac-Promotor (De Boer et al. 1983), gefolgt von einem DNA-Fragment mit Mehrfach-Klonierschnittstellen und schließlich den T1T2-Terminator des rrnB-Gens von Escherichia coli (Brosius et al. 1981). Durch Verwendung des T1T2-Terminators wird die stabile Replikation des Plasmids pZ8-1 in C.glutamicum gesichert. Escherichia coli DH5/pZ8-1 wurde bei der Deutschen Stammsammlung von Mikroorganismen unter DSM 4938 hinterlegt. Die Eignung des Plasmids pZ8-1 als Expressionsvektor wurde durch die Insertion des Phosphoenolpyruvat-Carboxylase-Gens von Corynebacterium glutamicum ATCC 13032 (Britische Patentanmeldung 8821319.4) in die Mehrfach-Klonierschnittstelle gezeigt. Das dadurch entstandene Plasmid pDM7 (Abb. 11) hat eine Länge von 10,4 kb und bewirkt in Corynebacterium glutamicum eine etwa 20fach gesteigerte Expression des Enzyms Phosphoenolpyruvat-Carboxylase.

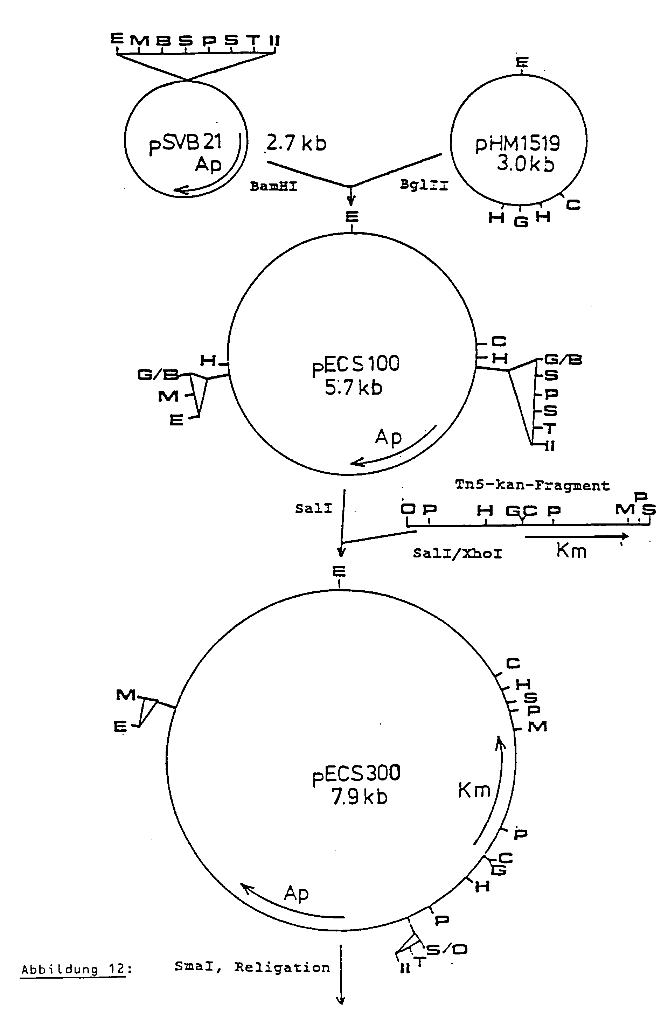

Die Konstruktion des Plasmids pDM7 gelingt dem Fachmann nach bekannten Methoden unter Verwendung der hinterlegten Plasmide pZ8-1 (DSM 4938) und pDM6 (DSM 4242), das das ppc-Gen enthält (Abb. 12)

Gegenstand der Erfindung sind ebenfalls Mikroorganismen der Gattungen Escherichia, Corynebacterium, Brevibacterium, insbesondere der Aminosäuren-ausscheidenden Mutanten, die die erfindungsgemäß mutierte DNA oder Plasmide und Resistenzgene von C. xerosis M82B inseriert enthalten.

### Beispiele

1. Isolierung und Charakterisierung eines Resistenzplasmids aus *Corynebacterium xerosis*
Resistenzplasmid-tragende Stämme von *C.xerosis* wurden erstmalig 1983 beschrieben (Kono et al.). Die Autoren untersuchten dabei klinische Isolate und fanden bei einigen Stämmen von *C.xerosis* mit einem breiten Resistenzspektrum große (>40 kb) Plasmide. Das R-Plasmid des Stammes M82B (pCXM82B), das Resistenzen gegen Erythromycin, Chloramphenicol, Kanamycin und Tetracyklin trägt wurde in Rahmen der Erfindung näher charakterisiert.
1.1 Modifizierte Lyse zur Gewinnung von Plasmid-DNA
Die Lysemethode nach Birnboim und Doly (1982) mit den Modifikationen für *C.glutamicum* (Thierbach et al. 1988) ist auf *C.xerosis* nur bedingt anwendbar. Aus diesem Grund wurde die folgende Vorbehandlung, die auf einer Aceton-Behandlung nach Heath et al. (1986) beruht, für eine Lyse von *C.xerosis* nach Birnboim und Doly entwickelt.
10 Milliliter (ml) einer Übernachtkultur von *C.xerosis* in LBG (Luria Broth mit 2g/l Glucose - Maniatis et al.1982) mit zugesetztem Antibiotikum werden zu 200 ml LBG mit Antibiotikum und 10% Glycin gegeben und 20 Stunden bei 30°C im Schüttler (120 U/min) gewachsen. Die Zellen werden durch Zentrifugation (10 Minuten bei 6000 U/min) in der Zentrifuge Beckman J2-21 (Rotor JA14) geerntet und in 1 ml TES (50mM Tris, 5 mM EDTA, 50mM NaCl, pH 8.0) aufgenommen. Nach Zugabe von 40 ml Aceton werden die Zellen 6 Minuten in Eis inkubiert und dabei in Abständen von 30 Sekunden durch Schütteln vermischt. Danach werden die Zellen pelletiert und zweimal mit je 40 ml TES gewaschen. Nach erneuter Zentrifugation wird das Pellet in 40 ml FL-Puffer (410mM Saccharose, 10mM MgCl, 50% MMYC-Medium - Katsumata et al.1984) mit 20 mg/ml Lysozym aufgenommen und mehrfach durch eine 50ml-Plastik-Spritze (Fresenius) gepresst. Das so hergestellte Homogenisat wird unter Schütteln (100 rpm) 5 Stunden bei 37°C inkubiert und dann abzentrifugiert. Die folgenden Schritte der Prozedur entsprechen dann der Lyse nach Birnboim und Doly (1982) ohne Lysozymbehandlung. Die mit 96% Äthanol ausgefällte Plasmid-DNA wird anschließend in 150 »l TE aufgenommen und die DNA-Konzentration bei 20 nm im Photometer bestimmt. Auf diese Weise werden aus 200 ml stationärer Kultur von *C.xerosis* etwa 20 »g pCXM82B-DNA gewonnen.
1.2 Restriktionskartierung des Plasmids pCXM82B Zur Kartierung des aus *C.xerosis* isolierten Plasmids
pCXM82B wurden Restriktionsenzyme eingesetzt, die dieses etwa 50 Kilobasen (kb) große Plasmid relativ selten schneiden. Die unten genannten Enzyme (Tabelle 1a) haben zwischen einer und vier Erkennungsstellen auf dem Plasmid.

**Tabelle 1a**

| Zur Grobkartierung von pCXM82B eingesetzte Enzyme | |
|---|---|
| Restriktionsenzym | Anzahl der Schnittstellen |
| *Cla*I | 3 |
| *Eco*RV | 3 |
| *Hpa*I | 1 |
| *Nsi*I | 4 |

Alle Restriktionsverdauungen erfolgten nach den Angaben der Hersteller. Durch Doppelverdauungen konnte die Lage der Schnittstellen relativ zueinander festgestellt werden.
Eine genaue Kartierung erfolgte nach Klonierung überlappender Restriktionsfragmente des Plasmids, die mit häufiger schneidenden Enzymen (Tabelle 1b) zuerst in den *E.coli* Plasmidvektor pUC19 (Norrander et al.1983) kloniert, dann kartiert und später zu einer zirkulären Karte des Plasmids vereinigt wurden. Dabei lieferte die Grobkartierung die Anhaltspunkte zum Erstellen der genauen Karte (Abb.1). Der Stamm *Corynebacterium xerosis* M82B mit dem R-Plasmid pCXM82B wurde unter der Bezeichnung DSM 5021 bei der Deutschen Stammsammlung von Mikroorganismen hinterlegt.

**Tabelle 1b**

| Zur Subklonierung und Feinkartierung von pCXM82B eingesetzte Enzyme | |
|---|---|
| Restriktionsenzym | Anzahl der Schnittstellen |
| *Bam*HI | 7 |
| *Bgl*II | 8 |
| *Eco*RI | 10 |
| *Hind*III | 8 |
| *Kpn*I | 5 |
| *Pst*I | 13 |
| *Sal*I | 13 |
| *Xba*I | 2 |

Alle Längenbestimmungen erfolgten durch Gelelektrophorese in Agarosegelen und Vergleich mit Längenstandards (DNA des Bakteriophagen λ, verdaut mit *Eco*RI und *Hin*dIII bzw. *Pst*I).
2. Klonierung von Antibiotika-Resistenzgenen aus *C.xerosis* in *E.coli*
Zur Isolierung von DNA-Fragmenten mit Antibiotika-Resistenz-Eigenschaften wurden die überlappenden Subklone im *E.coli*-Plasmid pUC19 (siehe 1.2) genutzt.
2.1 Klonierung des Chloramphenicol-Resistenzgens
   DNA des Plasmids pCXM82B wurde nach der in 1.1 beschriebenen Methode aus *C.xerosis* isoliert und mit dem Restriktionsenzym *Bgl*II gespalten. Aus *E.coli* nach bekannten Verfahren (Maniatis et al. 1982) gewonnene Plasmid-DNA des Vektors pUC19 wurde mit *Bam*HI verdaut und mit alkalischer Phosphatase behandelt. Bei Plasmide wurden gemischt und das Gemisch mit T4-DNA-Ligase behandelt. Mit diesem Gemisch wurde dann *E.coli* JM83 (Messing 1979) transformiert. Es konnte aus den Transformanten ein Plasmid isoliert werden, welches Resistenz gegen das Antibiotikum Chloramphenicol (25 »g/ml auf Agarplatten des Antibiotic Medium No.3-Firma Oxoid) verleihen. Dieses Plasmid, genannt pBg12, ist neu und besteht aus dem pUC19-Vektor mit einem 5 kb langen Insert von pCXM82B-DNA (Abb. 2a). Das Plasmid pBg12 wurde mit den Enzymen *Bam*HI und *Pst*I behandelt. Die Verdauung mit *Pst*I wurde partiell durchgeführt. Das aus E.coli JM83 isolierte Plasmid pSVB21 (Arnold und Pühler, 1988) wurde ebenfalls mit *Bam*HI und *Pst*I verdaut. Beide wurden anschließend gemischt und das Gemisch mit T4-DNA-Ligase behandelt. Nach Transformation von E.coli JM83 wurde das Plasmid pCX10 isoliert. Dieses Plasmid ist neu und trägt im Vektor pSVB21 ein 1.9 kb großes *Bam*HI-*Pst*I-Subfragment des Inserts von pBg12 (Abb.2a).
2.2 Klonierung des Kanamycin-Resistenzgens
   Plasmid-DNA von pCXM82B wurde mit dem Enzym *Eco*RV verdaut und mit *Sma*I-gespaltener und mit alkalischer Phosphatase behandelter pUC19-DNA gemischt. Das Gemisch wurde mit T4-DNA-Ligase inkubiert und nach *E.coli* JM83 transformiert. Aus einer Kanamycin-resistenten Transformante wurde das Plasmid pEVK1 isoliert, das dem Stamm JM83 eine Resistenz gegen 25 »g/ml Kanamycin und 10 »g/ml Neomycin (Antibiotic Medium No.3) verleiht. pEVK1 ist neu und besteht aus dem Plasmid pUC19 mit einem 2.7 kb langen DNA-Fragment aus pCXM82B (Abb. 2b).
2.3 Klonierung des Erythromycin-Resistenzgens
   Nach der in 1.1 beschriebenen Methode isolierte Plasmid-DNA von pCXM82B wurde mit dem Enzym *Sal*I gespalten und mit dem ebenfalls *Sal*I-gespaltenen und mit alkalischer Phosphatase behandelten Plasmid pUC19 gemischt. Das Gemisch wurde mit T4-DNA-Ligase behandelt und nach *E.coli* JM83 transformiert. Es wurde eine Transformante isoliert, die auf Antibiotic Medium No.3 resistent gegen 120 »g/ml Erythromycin war und nach dieser Primärselektion eine Resistenz von mehr als 2mg/ml aufwies. Dieses Plasmid (pSalE2) ist neu und besteht aus einem 8.5 kb langen Insert im Vektor pUC19 (Abb. 2c). Die auf diesem DNA-Fragment lokalisierte Erythromycin-Resistenz ist durch geringe Mengen (10-100»g/ml) an Erythromycin induzierbar.
2.4 Expression der Resistenzgene in *E.coli*
   Die in den *E.coli*-Vektor pUC19 klonierten Antibiotika-Resistenz-vermittelnden DNA-Fragmente von pCXM82B (siehe 2.) wurden auf ihre minimale Hemmkonzentration hin untersucht (Tabelle 2). Dazu wurden die im *E.coli*-Stamm JM83 vorliegenden Klone in LBG-Flüssigmedium aus einer Antibiotika-enthaltenden Vorkultur mit einer Konzentration von etwa 10⁶ Zellen pro Milliliter angeimpft, verschiedene Konzentrationen der entsprechenden Antibiotika zugesetzt und ca. 16 Stunden bei 37°C inkubiert.

3. DNA-Sequenzanalyse des Chloramphenicol-Resistenzgens
Die Nukleotidsequenz des 1.9kb langen *Bam*HI-*Pst*I-DNA-Fragments wurde nach der Methode von Maxam und Gilbert (1977) mit den Modifikationen von Arnold und Pühler (1988) und nach der Methode von Sanger et al. (1978) vollständig bestimmt. Die Subklonierung erfolgte dabei in die *E.coli*-Sequenziervektoren pSVB20, pSVB21, pSVB25 und pSVB27 (Arnold und Pühler 1988). Die Sequenzierstrategie ist in Abb.3a wiedergegeben. Das DNA-Stück trägt Restriktionsschnittstellen für die Enzyme *Bam*HI, *Eco*RI, *Nru*I, *Pst*I, *Sac*I und *Sma*I mit denen auch die Subklone hergestellt wurden.
Die Sequenz beider DNA-Stränge wurde mit Hilfe des Sequenzanalyse-Programmpaketes ANALYSEQ (Staden 1986) analysiert. Die Kodierbereichsanalyse (Abb.3b) gibt einen für ein Protein codierenden Bereich zwischen den Positionen 520 und 1720 an. In der Nukleotidsequenz des 1.9kb DNA-Fragments (Abb.4) findet sich dort ein langes offenes Leseraster. Es beginnt an der Position 545 mit dem Startcodon ATG und endet an der Position 1717 mit 2 aufeinander folgenden Stopcodons (TGA). Sechs Nukleotide vor dem Startcodon befindet sich eine Ribosomenbindungsstelle (GGAG). Das Molekulargewicht des Proteins beträgt 39.3 Mdals. Es hat keinerlei Sequenzhomologie zu den bekannten Chloramphenicol-Acetyltransferase-Genen von anderen Organismen (Stand EMBL DATA Library Release 15).

**Tabelle 2**

| Minimale Hemmkonzentrationen (MIC) der klonierten Resistenzen | | | | |
|---|---|---|---|---|
| Stamm | Plasmid | Kanamycin | Chloramphenicol | Erythromycin |
| *C.xerosis* M82B | pCXM82B | >1000 | >200 | >2000 |
| *E.coli* JM83 | - | 20 | 5 | 300 |
| *E.coli* JM83 | pUC19 | 20 | 5 | 300 |
| *E.coli* JM83 | pEVK1 | >1000 | n.t. | n.t. |
| *E.coli* JM83 | pBg12 | n.t. | >100 | n.t. |
| *E.coli* JM83 | pSalE2 | n.t. | n.t. | >2000 |

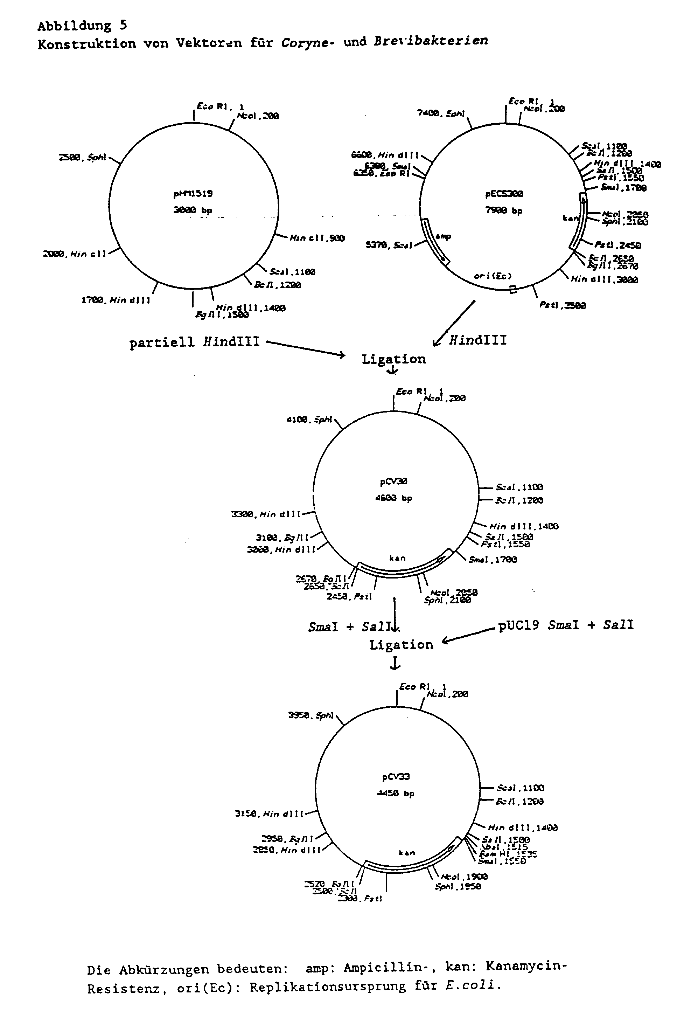

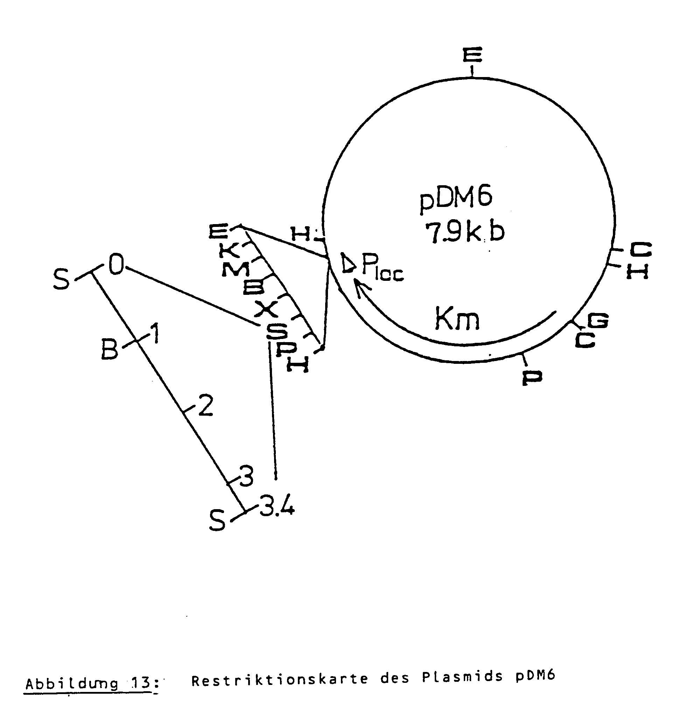

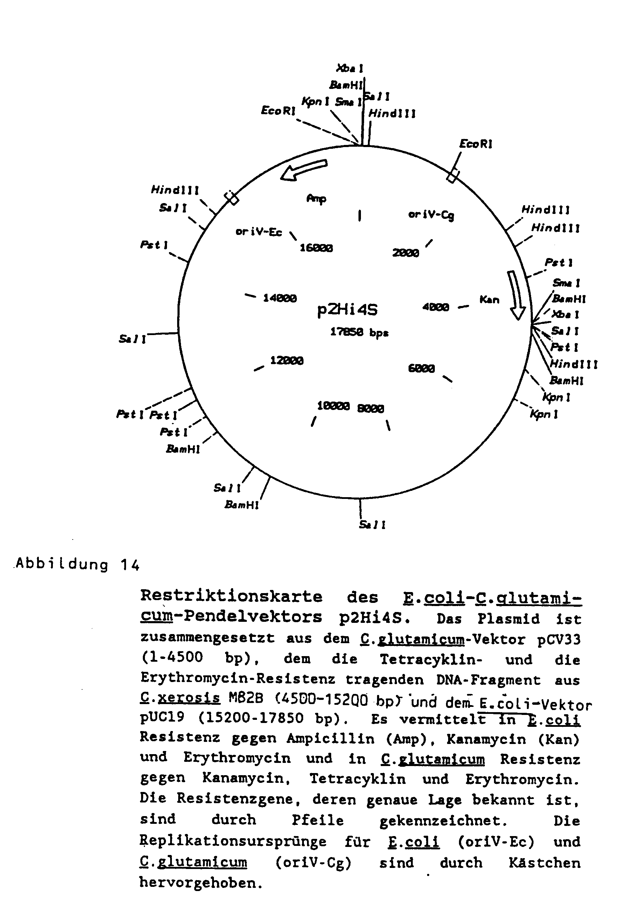

Alle Werte sind in »g/ml angegeben.
4. Konstruktion von Plasmidvektoren (pCV30, pCV33) für *Coryne*-und *Brevibakterien* und Herstellung von mutierten Formen des Replikons pHM1519 und des Tn*5*-Kanamycin-Resistenzgens
4.1 Konstruktion von Vektorplasmiden für *Coryne*- und *Brevibakterien*
   Die Konstruktion der Vektoren ist in Abb.5 wiedergegeben. Der in der Britischen Patentanmeldung 8821319.4 beschriebene Pendelvektor pECS300 (Abb. 13) wurde aus *E.coli* JM 83 isoliert und nach *C.glutamicum* ATCC 13032 transformiert (Thierbach et al. 1988). Das Plasmid pHM1519 (Miwa et al. 1984) wurde partiell mit *Hin*dIII restringiert und mit dem vollständig *Hin*dIII-verdauten pECS300 ligiert. Das Ligationsgemisch wurde nach *C.glutamicum* transformiert, von 24 der Transformanden Plasmid-DNA isoliert und mit *Hin*dIII gespalten. In den kürzesten dieser Plasmide lag das die Kanamycin-Resistenz tragende *Hin*dIII-Fragment in eine der *Hin*dIII-Schnittstellen des kryptischen Replikons pHM1519 kloniert vor (pCV30). Das 4.5 kb große Plasmid pCV30 wurde dann mit *Sma*I und *Sal*I gespalten und ein etwa 25 bp langes *Sma*I-*Sal*I-Stück aus dem *E.coli*-Vektor pUC19 (Norrander et al. 1983) inseriert. Das resultierende Plasmid pCV33 ist 4.5kb lang und trägt einzelne Schnittstellen für die Enzyme *Sma*I, *Bam*HI, *Xba*I und *Sal*I.
4.2 *In vitro*-Mutagenese von Plasmiden aus *Corynebacterium glutamicum*
   Um Restriktionsschnittstellen in klonierten Antibiotika-Resistenzgenen zur Insertionsinaktivierung nutzen zu können, ist es unter Umständen nötig, zuerst endogene Schnittstellen im Plasmid zu entfernen. Dies kann z.B. durch Auffüllen der Schnittstelle mit Nukleosidtriphosphaten oder durch Nuklease-Behandlung erfolgen. Diese Methoden sind aber nur sehr bedingt anwendbar, wenn es sich um eine in einem Gen oder in einer anderen essentiellen Region gelegene Schnittstelle handelt, da ein Wechsel des Leserasters oder eine Deletion von DNA erreicht wird. Eine Methode, die Punktmutationen setzt, wie die Mutagenese mit Hydroxylamin (Ashley et al. 1985) birgt aber die Gefahr, unspezifisch mehrere Mutationen verteilt über ein ganzes Plasmid zu erzeugen. Die hier entwickelte modifizierte Hydroxylamin-Mutagenese zeichnet sich vor allem durch eine hohe Ortsspezifität aus. Dies wird durch Linearisierung der Plasmide mit den entsprechenden Enzym und durch niedrigere Temperaturen als beschrieben erreicht, so daß der DNA-Doppelstrang nur im Bereich der Schnittstelle oder überhaupt nicht aufschmilzt. Sowohl die *Eco*RI-Schnittstelle in 4.2.1 (pHM1519 - Miwa et al.1984) als auch die *Pst*I-Schnittstelle im Kanamycin-Resistenzgen liegen in essentiellen Bereichen und konnten weder durch Auffüllen noch durch S1-Nukleasebehandlung entfernt werden. Eine Punktmutation (Cytosin --> Thymin) im Kanamycin-Resistenzgen würde aber sowohl das Leseraster als auch die Aminosäuresequenz des Proteins erhalten.
   4.2.1 Mutagenese der *Eco*RI-Schnittstelle im Replikon von pCV33 und Erzeugung von pCV34
      Das Plasmid pCV33 wurde nach Thierbach et al. (1988) isoliert und mit dem Enzym *Eco*RI gespalten. Der Mutageneseansatz besteht aus etwa 2»g Plasmid-DNA in 60»l TE-Puffer, 180»l 1.5M Hydroxylamin-HCl in 25mM EDTA, 5»l 0.25M EDTA und 13»l 1M Tris-HCl, pH 8.0. Der Ansatz wurde gemischt und 30 Minuten bei 68°C inkubiert. Nach Phenol-Behandlung und Äthanol-Fällung der Plasmid-DNA (Maniatis et al. 1982) wurde das DNA-Pellet in 20 »l TE aufgenommen und mit T4-DNA-Ligase behandelt. Nach Transformation von *C.glutamicum* ATCC 13032 mit der Plasmid-DNA konnten Transformanten isoliert werden, die durch das Enzym *Eco*RI nicht mehr gespalten werden. Das Plasmid pCV34 (Abb.6a) ist neu. Der Stamm *Corynebacterium glutamicum* ATCC 13032 mit dem Plasmid pCV34 wurde bei der Deutschen Stammsammlung von Mikroorganismen unter DSM 5025 hinterlegt.
   4.2.2 Mutagenese der *Pst*I-Schnittstelle im Kanamycin-Resistenzgen von pCV34 und Erzeugung des Plasmids pCV36
      Analog zur oben genannten Vorgehensweise (4.2.1) wurde pCV34-Plasmid-DNA mit dem Enzym *Pst*I linearisiert und der Hydroxylamin-Mutagenese unterzogen. Die Reaktionsdauer und Temperatur betrugen dabei 20 Minuten und 68°C. Nach Ligation mit T4-DNA-Ligase und Transformation nach *C.glutamicum* konnte das Plasmid pCV36 (Abb.6b) isoliert werden, das gegen Restriktion durch *Eco*RI und *Pst*I resistent ist. pCV36 ist neu und weist die gleiche Kanamycin-Resistenzhöhe auf wie pCV33. *C.glutamicum* ATCC 13032/pCV36 wurde bei der Deutschen Stammsammlung von Mikroorganismen unter DSM 5026 hinterlegt.

5. Konstruktion von Plasmiden mit zwei Antibiotika-Resistenzgenen und der Möglichkeit zur Insertionsinaktivierung
5.1 Herstellung des Plasmids pCVX4 unter Verwendung des Chloramphenicol-Resistenzgens aus *C.xerosis*
Aus dem *E.coli*-Plasmid pCX10 (2.1), welches ein 1.9 kb großes Insert von pCXM82B-DNA trägt (Abb.2a), wurde ein 1.9 kb langes *Bam*HI-*Sal*I-Fragment isoliert. Die *Sal*I-Schnittstelle stammt dabei nicht aus dem *C.xerosis*-DNA-Fragment, sondern befindet sich unmittelbar neben der das DNA-Fragment begrenzenden *Pst*I-Schnittstelle im Vektor pSVB21. Das isolierte DNA-Stück wurde mit dem mit *Bam*HI und *Sal*I restringierten und mit alkalischer Phosphatase behandelten Plasmidvektor pCV36 gemischt. Dieses Gemisch wurde ligiert und nach *C.glutamicum* transformiert. Aus einer Choramphenicol-resistenten Transformante konnte das Plasmid pCVX2.1 isoliert werden (Abb.7). Das neue Plasmid wurde dann mit *Sal*I linearisiert und mit der Nuklease *Bal*31 behandelt. Nach der Behandlung mit T4-DNA-Polymerase und Ligation mit T4-DNA-Ligase wurde *C.glutamicum* mit dem Plasmid-Gemisch transformiert und aus einer Transformante das Plasmid pCVX4 isoliert. pCVX4 ist neu, 6.5 kb lang und vermittelt der Zelle Resistenzen gegen Kanamycin und Chloramphenicol. Es hat die Schnittstelle für *Sal*I und eine der *Pst*I-Erkennungsstellen verloren und bietet Insertionsinaktivierungsmöglichkeiten durch die Enzyme *Eco*RI, *Pst*I und *Mlu*I im Cm-Gen (Abb.7). Der das Plasmid pCVX4 tragende Stamm *C.glutamicum* ATCC 13032 wurde bei der Deutschen Stammsammlung von Mikroorganismen unter DSM 5022 hinterlegt.
5.2 Herstellung des Plasmids pCVX10 unter Verwendung des Kanamycin-Resistenzgens aus *C.xerosis*
Das Plasmid pCVX2.1 (siehe 5.1) wurde aus *C.glutamicum* isoliert, mit *Bam*HI und *Bgl*II verdaut, mit T4-DNA-Ligase behandelt und nach *C.glutamicum* transformiert. Aus einer Kanamycin-sensitiven und Chloramphenicol-resistenten Transformante wurde das Plasmid pCVX2.1ΔBB isoliert (Abb.8). Dieses weist eine Deletion von 1.4 kb gegenüber pCVX2.1 auf, die das ganze Kanamycin-Resistenzgen von Tn*5* umfasst. pCVX2.1ΔBB wurde mit *Sal*I linearisiert, mit alkalischer Phosphatase behandelt und mit dem ebenfalls mit *Sal*I-gespaltenen Plasmid pCXM82B gemischt. Nach Ligation und Transformation wurde aus einer Kanamycin-resistenten Transformante das Plasmid pCVX10 isoliert (Abb.8a). pCVX10 ist neu, 7 kb groß und trägt Resistenzen gegen Kanamycin und Chloramphenicol. Das Plasmid bietet zusätzlich zum Cm-Gen neue Insertionsinaktivierungsmöglichkeiten durch die Enzyme *Xho*I und *Cla*I im Kanamycin-Resistenzgen. Der das Plasmid pCVX10 tragende Stamm *C.glutamicum* ATCC 13032/pCVX10 wurde bei der Deutschen Stammsammlung von Mikroorganismen unter DSM 5023 hinterlegt.
5.3 Herstellung des Plasmids pCVX15 unter Verwendung des Erythromycin-Resistenzgens aus *C.xerosis*
Das Plasmid pCVX2.1ΔBB (5.2) wurde mit dem Restriktionsenzym *Sal*I gespalten und mit alkalischer Phosphatase behandelt. pSalE2 (siehe 2.3) wurde mit *Sal*I restringiert und beide Plasmide miteinander gemischt. Das Gemisch wurde mit T4-DNA-Ligase behandelt und nach *C.glutamicum* transformiert. Aus einer gegen 10 »g/ml Erythromycin resistenten Transformante wurde das Plasmid pCVX15 isoliert (Abb.8b), welches das 8.5 kb lange, in *E.coli* ebenfalls Em-Resistenz-vermittelnde, DNA-Fragment trägt. Das Plasmid pCVX15 ist neu und trägt singuläre Schnittstellen für die Enzyme *Bgl*II, *Bam*HI und *Xba*I auf dem die Erythromycin-Resistenztragenden DNA-Fragment und für *Eco*RI und *Pst*I im Cm-Gen. Der das Plasmid pCVX15 tragende Stamm *C.glutamicum* ATCC 13032/PCVX15 wurde bei der Deutschen Stammsammlung von Mikroorganismen unter DSM 5024 hinterlegt.
5.4 Austausch von mutierten DNA-Fragmenten in Plasmidvektoren - Konstruktion des Plasmids pZ9
Das Plasmid pZ1, welches in der Deutschen Patentanmeldung 3737729.9 beschrieben ist, wurde aus *E.coli* nach bekannten Methoden (Maniatis et al. 1982) isoliert. Nach Linearisierung mit der Restriktionsendonuklease *Pst*I wurde das Plasmid mit Hilfe der Nuklease *Bal*31 um etwa eine Kilobase (kb) verkürzt. Nach Behandlung mit T4-DNA-Ligase und Transformation von *E.coli* DH5 (Hanahan 1985) konnte ein als pZ2-1 bezeichnetes Plasmid isoliert werden. Nach Verdauung des Plasmids pCV34 mit *Hin*cII und *Pst*I wurde das 2.4 kb lange *Hin*cII-DNA-Fragment mittels Elektroelution isoliert. Das so gewonnene Fragment wurde mit dem Plasmid pZ2-1, das mit *Hin*cII und alkalischer Phosphatase behandelt worden war, gemischt und das Gemisch mit T4-DNA-Ligase behandelt. Nach Transformation von *E.coli* DH5 wurde aus einer Kanamycin-resistenten Transformante das Plasmid pZ3-4 isoliert, welches durch die Restriktionsendonuklease *Eco*RI nicht mehr gespalten wird.
Das Plasmid pCVX2.1, welches das Chloramphenicol-Resistenzgen aus *C.xerosis* trägt (siehe 5.1), wurde mit *Bam*HI und *Sal*I behandelt. Das 1.9 kb lange DNA-Fragment konnte anschließend durch Behandlung mit *Bal*31 um etwa 0.1 kb verkürzt werden. Nach Behandlung mit T4-DNA-Polymerase wurde das beschriebene DNA-Fragment in die ScaI-Schnittstelle von pZ3-4 inseriert, wobei der als pZ9 bezeichnete Vektor entstand (Abb.9). *E.coli* DH5α/pZ9 wurde bei der Deutschen Sammlung von Mikroorganismen unter DSM 4939 hinterlegt. Das Plasmid pZ9 ist neu und trägt singuläre Restriktionsschnittstellen für *Xho*I und *Cla*I im Kanamycin-Resistenzgen und für *Pst*I, *Mlu*I und *Eco*RI in Chloramphenicol-Resistenzgen, die zur Insertionsinaktivierung genutzt werden können.
5.6. Expression der Gene für Kanamycin-, Chloramphenicol-, Erythromycin- und Tetrazyklin-Resistenz aus C.xerosis in C.glutamicum
Die zuvor konstruierten Plasmidvektoren wurden nach Transformation (Thierbach et al. 1988) in C.glutamicum ATCC 13032 auf ihre minimalen Hemmkonzentrationen getestet (Tabelle 3).

**Tabelle 3**

| Minimale Hemmkonzentrationen (MIC) der klonierten Resistenzen | | | | | |
|---|---|---|---|---|---|
| Stamm | Plasmid | Kanamycin | Chloramphenicol | Erythromycin | Tetrazyklin |
| Cx | pCXM82B | >1000 | >200 | >2000 | 40 |
| Cg | - | <10 | <3 | <5 | <3 |
| Cg | pCV34 | >500^{*1} | n.t. | n.t. | n.t. |
| Cg | pCVX10 | >1000 | >80 | n.t. | n.t. |
| Cg | pCVX4 | >500^{*1} | >80 | n.t. | n.t. |
| Cg | pCVX15 | n.t. | >80 | >1000 | n.t. |
| Cg | pZ9 | >500^{*2} | >80 | n.t. | n.t. |

Die minimale Hemmkonzentration wurde in LBG-Medium analog zu 2.4 ermittelt. Cx bedeutet C.xerosis M82B und Cg C.glutamicum ATCC 13032. Die Kanamycin-Resistenz der Plasmide pCV34 und pCVX4 (*¹) stimmt vom Tn5 und die des Plasmids pZ9 von Tn903 (*²). Alle Werte sind in »g/ml angegeben.
6. Konstruktion von Expressionsvektoren für Coryne- und Brevibakterien mit Hilfe des mutierten pHM1519-Replikons
6.1 Konstruktion des Expressionsplasmids pZ8-1 mit dem *tac*-Promoter und dem *rrn*B-T1T2-Terminator
   Der Expressionsvektor pKK223-3 (Brosius 1984) wurde von der Firma Pharmacia bezogen und mit den Enzymen *Sca*I und *Bam*HI behandelt. Die Behandlung mit *Bam*HI wurde dabei als partielle Verdauung durchgeführt. Das entstehende 1.1 kb lange *Sca*I-*Bam*HI-Fragment, das den *tac*-Promotor, eine DNA-Sequenz mit Erkennungsstellen für die Enzyme *Pst*I, *Sal*I, *Bam*HI und *Eco*RI und den T1T2-Terminator des *rrn*B-Gens trägt, wurde mittels Elektroelution isoliert und mit T4-DNA-Polymerase behandelt. Das beschriebene Fragment wurde mit dem Plasmid pZ3-4, das mit *Sca*I linearisiert worden war, gemischt, mit T4-DNA-Ligase behandelt und *E.coli* DH5 mit dem Ligationsgemisch transformiert. Aus einer Kanamycin-resistenten Transformante wurde das Plasmid pZ8-1 isoliert, das in Abb.10 dargestellt ist. *E.coli* DH5/pZ8-1 wurde bei der Deutschen Stammsammlung von Mikroorganismen unter der Nummer DSM 4938 hinterlegt. Nach Transformation von *C.glutamicum* ATCC 13032 konnte die Replikationsfähigkeit von pZ8-1 nachgewiesen werden. Das Plasmid pZ8-1 vermehrt sich unter nichtselektiven Kulturbedingungen für mindestens 70 Generationen stabil in *Corynebacterium glutamicum*.
6.2 Überexpression des PEP-Carboxylase-Gens (*ppc*) von *C.glutamicum* mit Hilfe des Vektors pZ8-1
   Das Plasmid pDM2, welches in der Britischen Patentanmeldung 8821319.4 beschrieben ist, wurde mit *Sal*I und *Sma*I behandelt und mit dem Vektor pZ8-1 gemischt, der mit *Sal*I linearisiert worden war. Das DNA-Gemisch wurde mit T4-DNA-Ligase behandelt und das Ligationsgemisch zur Transformation von *E.coli* XH11 (Mountain et al. 1984) verwendet Aus einer Kanamycin-resistenten und Succinat-prototrophen Transformante wurde das in Abb.11 dargestellte Plasmid pDM7 isoliert. *C.glutamicum* ATCC 13032 wurde mit dem Plasmid pDM7 transformiert. Nach Anzucht in MMYE-Medium (Katsumata et al. 1984) wurde, wie in der Britischen Patentanmeldung 8821319.4 beschrieben, der spezifische PEP-Carboxylase-Gehalt in der Transformante ATCC 13032/pDM7 und im Kontrollstamm ATCC 13032/pZ8-1 bestimmt.
   Der spezifische Gehalt an PEP-Carboxylase betrug bei Stamm ATCC 13032/pZ8-1 0.16 U/mg Protein und bei Stamm ATCC 13032/pDM7 3.00 U/mg Protein.

### Literatur

Arnold und Pühler (1988) Gene 70, 171ff
Birch et al. (1985) J. Gen. Microbiol. 131, 1299ff
Birnboim und Doly (1982) Nucl. Acids Res. 7, 1513ff
Bolivar et al. (1979) Life Sciences 25, 807ff
Brosius et al. (1981) J. Mol. Biol. 148, 107ff
Brosius (1984) Gene 27, 161ff
Chang et al. (1978) J. Bact. 134, 1141ff
De Boer et al. (1983) Proc. Natl. Acad. Sci. USA 78, 21ff
Hanahan (1985) in Glover (ed.) DNA cloning Vol.1, IRL Press
Heath et al. (1986) Appl. Environ. Microbiol. 51, 1138ff
Katsumata et al. (1984) J. Bacteriol. 159, 306ff
Kono et al. (1983) Antimicrob. Agents Chemother. 23, 506ff
Maniatis et al. (1982) Molecular cloning, Cold Spring Harbor Lab.
Maxam und Gilbert (1980) Methods Enzymol. 65, 499ff
Messing (1979) Recomb. DNA Techn. Bull., NIH Publ. 79-99, 2, 43ff
Miwa et al. (1984) Agric. Biol. Chem. 48, 2901ff
Morinaga et al. (1987) J.Biotech. 5, 305ff
Mountain et al. (1984) Mol. Gen. Genet. 197, 82ff
Norrander et al. (1983) Gene 26, 101ff
Sanger et al. (1977) Proc. Natl. Acad. Sci. USA 74, 5463ff
Santamaria (1984) J. Gen. Microbiol. 130, 2237ff
Santamaria (1987) Gene 56, 199ff
Staden (1986) Nucl. Acids Res. 14, 217ff
Thierbach et al. (1988) Appl. Microbiol. Biotechnol. 29, 356ff

## Patentansprüche

1. Verfahren zur ortspezifischen Mutagenese von DNA an den Restriktionsschnittstellen
dadurch gekennzeichnet, daß man die DNA isoliert, mit einem geeigneten Restriktionsenzym spaltet, die so behandelte DNA mit einem Hydroxylamin enthaltenden Mutageneseansatz mischt und bei erhöhter Temperatur inkubiert mit der Maßgabe, daß der DNA-Doppelstrang nicht oder nur im Bereich der Schnittstelle "aufschmilzt", anschließend die DNA nach an sich bekannten Methoden aufnimmt , mit einer Ligase behandelt, einen geeigneten Mikroorganismus mit der so mutierten DNA transformiert und die Transformanten isoliert.

2. Verfahren gemäß Anspruch 1,
dadurch gekennzeichnet, daß es sich bei der DNA um ein Resistenzgen und / oder das Replikon handelt.

3. Verfahren gemäß Anspruch 1,
dadurch gekennzeichnet, daß es sich bei der DNA um ein Plasmid handelt.

4. Verfahren gemäß Anspruch 2,
dadurch gekennzeichnet, daß der oder die für eine oder mehrere Resistenzen codierende(n) DNA-Abschnitt(e) (Resistenzgen(e)) von einem Transposon stammt(en).

5. Verfahren gemäß Anspruch 2,
dadurch gekennzeichnet, daß der oder die für eine oder mehrere Resistenzen codierende(n) DNA-Abschnitt(e) aus Corynebacterium xerosis M82B stammt(en).

6. Plasmid pCV34,
gekennzeichnet durch die in Abb. 6a wiedergegebene Restriktionskarte und hinterlegt in C.glutamicum unter der Bezeichnung DSM 5025.

7. Plasmid pCV36,
gekennzeichnet durch die in Abb. 6b wiedergegebene Restriktionskarte und hinterlegt in C.glutamicum unter der Bezeichnung DSM 5026.

8. Plasmidvektor pCVX4,
gekennzeichnet durch die in Abb. 7 wiedergegebene Restriktionskarte und hinterlegt in C.glutamicum unter der Bezeichnung DSM 5022.

9. Plasmidvektor pCVX10,
gekennzeichnet durch die in Abb. 8a wiedergegebene Restriktionskarte und hinterlegt in C.glutamicum unter der Bezeichnung DSM 5023.

10. Plasmidvektor pCVX15,
gekennzeichnet durch die in Abb. 8b wiedergegebene Restriktionskarte und hinterlegt in C.glutamicum unter der Bezeichnung DSM 5024.

11. Plasmid pZ9,
gekennzeichnet durch die in Abb. 9 wiedergebene Restriktionskarte und hinterlegt in E. coli unter der Bezeichnung DSM 4939.

12. Plasmid pZ8-1,
gekennzeichnet durch den tac- Promotor und den T1T2-Terminator des rrn8-Gens von E. coli und die in Abb. 10 wiedergegebene Restriktionskarte und hinterlegt in E. coli unter der Bezeichnung DSM 4938.

13. Plasmid pDM7,
gekennzeichnet durch die in Abb. 11 wiedergegebene Retriktionskarte und hergestellt durch Behandlung des Plasmids pDM2 mit SalI und SmaI, Mischung mit dem mit SalI linearisierten Vektor pZ8-1, Behandlung dieses Gemischs mit T4-DNA-Ligase, Transformation von E. coli XH11 mit dem Ligatiansgemisch und Isolierung aus einer Kanamycinresistenten und Succinat-prototrophen Transformante.

14. Verwendung von Plasmiden gemäß einem oder mehreren der Ansprüche 6 bis 13, zum Austausch von einem oder mehreren DNA-Segmenten, die mindestens eine oder mehrere Replikationsregion(en) und/oder Gene aufweisen, in Mehrkomponentenplasmiden gegen entsprechende DNA-Segmente die sich von den genannten durch das Vorhandensein einer oder mehrerer singulärer Restriktionsschnittstellen unterscheiden.

15. Verwendung von Plasmiden gemäß Anspruch 14,
dadurch gekennzeichnet, daß das oder die ausgetauschten Gen(e) der Zelle eine oder mehrere Wirkstoffresistenzen verleihen.

16. Verwendung von Plasmiden gemäß einem oder mehreren der Ansprüche 6 bis 13,
dadurch gekennzeichnet, daß in die Plasmide zusätzlich ein oder mehrere Resistenzgen(e) und gegebenenfalls ein Expressionssignal insertiert wird (werden).

17. Mikroorganismen der Gattungen Escherichia,
Corynebacterium oder Brevihacterium, insbesondere deren Aminosäuren ausscheidenden Mutanten, die DNA oder ein Plasmid gemaß einem oder mehreren der Ansprüche 6 bis 13 enthalten.

## Claims

1. A method of site-specific mutagenesis of DNA at the restriction cleavage sites, characterised in that the DNA is isolated and cleaved with a suitable restriction enzyme, the thus-treated DNA is mixed with a hydroxylamine-containing mutagenesis preparation and incubated at elevated temperature, taking care that the DNA double strand does not "melt" or only in the region of the cleavage site, after which the DNA is taken up by known methods and treated with a ligase and a suitable microorganism is transformed with the thus-mutated DNA and the transformants are isolated.

2. A method according to claim 1, characterised in that the DNA is a resistance gene and/or the replicon.

3. A method according to claim 1, characterised in that the DNA is a plasmid.

4. A method according to claim 2, characterised in that the DNA portion or portions (resistance gene or genes) which code for one or more resistances are derived from a transposon.

5. A method according to claim 2, characterised in that the DNA portion or portions which code for one or more resistances are derived from Corynebacterium xerosis M82B.

6. Plasmid pCV34, characterised by the restriction map given in Fig. 6a and deposited in C. glutamicum under the number DSM 5025.

7. Plasmid pCV36, characterised by the restriction map given in Fig. 6b and deposited in C. glutamicum under the number DSM 5026.

8. Plasmid vector pCVX4, characterised by the restriction map given in Fig. 7 and deposited in C. glutamicum under the number DSM 5022.

9. Plasmid vector pCVX10, characterised by the restriction map given in Fig. 8a and deposited in C. glutamicum under the number DSM 5023.

10. Plasmid vector pCVX15, characterised by the restriction map given in Fig. 8b and deposited in C. glutamicum under the number DSM 5024.

11. Plasmid pZ9, characterised by the restriction map given in Fig. 9 and deposited in E. coli under the number DSM 4939.

12. Plasmid pZ8-1, characterised by the tac promotor and the T1T2 terminator of the rrnB gene of E. coli and the restriction map given in Fig. 10 and deposited in E. coli under the number DSM 4938.

13. Plasmid pDM7, characterised by the restriction map given in Fig. 11 and prepared by treatment of plasmid pDM2 with SalI and SmaI, mixing with the vector pZ8-1 linearised with SalI, treatment of the mixture with T4-DNA ligase, transformation of E.coli XH11 with the ligation mixture and isolation from a kanamycin-resistant and succinate-prototrophic transformant.

14. Use of plasmids according to one or more of claims 6 to 13 for replacing one or more DNA segments containing at least one or more replication regions and/or genes in multicomponent plasmids by corresponding DNA segments which differ from the aforementioned segments by the presence of one or more unique restriction cleavage sites.

15. Use of plasmids according to claim 14, characterised in that the replaced gene or genes give the cell resistance to one or more active principles.

16. Use of plasmids according to one or more of claims 6 to 13, characterised in that one or more resistance genes and optionally an expression signal is (are) additionally inserted into the plasmids.

17. Micro-organisms of the genera Escherichia, Corynebacterium or Brevibacterium, more particularly mutants thereof which evolve amino acids and contain DNA or a plasmid according to one or more of claims 6 to 13.

## Revendications

1. Procédé de mutagenèse loco-spécifique de l'ADN sur des sites de section de restriction caractérisé en ce que l'on isole l'ADN que l'on clive avec un enzyme de restriction approprié, que l'on mélange l'ADN ainsi traité avec une fraction de mutagenèse contenant une hydroxylamine et que l'on fait incuber à température accrue, avec la restriction que le double brin d'ADN ne "fond" pas ou seulement dans la zone du site de section, que l'on reprend ensuite l'ADN selon les méthodes connues en soi, que l'on traite avec une ligase, que l'on transforme avec un microorganisme convenable avec l'ADN ainsi muté et que l'on isole le transformant.

2. Procédé selon la revendication 1, caractérisé en ce qu'il s'agit, pour l'ADN, d'un gène de résistance et/ou le replicon.

3. Procédé selon la revendication 1, caractérisé en ce qu'il s'agit, pour l'ADN, d'un plasmide.

4. Procédé selon la revendication 2, caractérisé en ce que l'un ou les segments d'ADN codant pour une ou plusieurs résistances, (gène(s) de résistance) découle(nt) d'un transposon.

5. Procédé selon la revendication 2, caractérisé en ce que l'un ou les segment(s) d'ADN codant pour une ou plusieurs résistances découle(nt) de Corynebactérium xerosis M82B.

6. Plasmide pCV34 caractérisé par la carte de restriction reproduite dans la figure 6a et déposé dans C.glutamicum, sous la désignation DSM 5025/

7. Plasmide pCV36 caractérisé par la carte de restriction reproduite dans la figure 6b et déposé dans C-glutamicum, sous la désignation DSM 5026.

8. Vecteur de plasmide pCVX4, caractérisé par la carte de restriction reproduite dans la figure 7 et déposé dans C.glutamicum, sous la désignation DSM 5022.

9. Vecteur de plasmide pCVX10, caractérisé par la carte de restriction reproduite dans la figure 8a et déposé dans C-glutamicum, sous la désignation DSM 5023.

10. Vecteur de plasmide pCVX15, caractérisé par la carte de restriction reproduite dans la figure 8b et déposé dans C.glutamicum, sous la désignation DSM 5024.

11. Plasmide pZ9, caractérisé par la carte de restriction reproduite dans la figure 9 et déposé dans E. coli sous la désignation DSM 4939.

12. Plasmide pZ8-1, caractérisé par le promoteur tac et l'agent de terminaison T1T2 du gène rrnB d'E.Coli et par la carte de restriction reproduite dans la figure 10, et déposé dans E. coli sous la désignation DSM 4938.

13. Plasmide pDM7, caractérisé par la carte de restriction reproduite dans la figure 11 et produit par traitement du plasmide pDM2 avec SalI et SmaI, par mélange avec le vecteur pZ8-1 linéarisé avec SalI, par traitement de ce mélange avec la T4-ADN-ligase, transformation d'E. coli XH11 avec le mélange de ligature et isolément de transformants, résistant à la kanamycine et succinatoprototrophes.

14. Utilisation de plasmides conformément à l'une ou plusieurs des revendications 6 à 13 pour l'échange d'un ou plusieurs segments d'ADN, qui possèdent au moins une ou plusieurs région(s) de réplication et/ou des gènes, dans des plasmides à plusieurs composants contre les segments correspondants d'ADN qui se différencient de ceux connus par la présence d'un ou plusieurs sites de section de restriction.

15. Utilisation des plasmides selon la revendication 14, caractérisée en ce que le ou les gènes échangés des cellules confèrent une ou plusieurs résistances contre des principes actifs.

16. Utilisation des plasmides selon l'une ou plusieurs des revendications 6 à 13, caractérisée en ce que l'on insère dans les plasmides en supplément un ou plusieurs gène(s) de résistance et le cas échéant un signal d'expression.

17. Micro-organismes des genres Escherichia, Corynebactérium ou Brevibactérium, en particulier leurs mutants qui sécrètent des aminoacides, renfermant de l'ADN ou un plasmide selon l'une ou plusieurs des revendications 6 à 13.
